# EUROPÄISCHE PATENTSCHRIFT

(11) **EP 1 729 646 B1**
(45) Veröffentlichungstag und Bekanntmachung des Hinweises auf die Patenterteilung: **27.11.2013**
(21) Anmeldenummer: 05730328.1
(22) Anmeldetag: 31.03.2005
(51) Int. Cl.: A61B 6/03

(54) **DREHÜBERTRAGER MIT DIELEKTRISCHEM WELLENLEITER**
ROTARY TRANSMITTER COMPRISING A DIELECTRIC WAVEGUIDE
DISPOSITIF DE TRANSMISSION ROTATIF À GUIDE D'ONDES DIÉLECTRIQUE

(30) Priorität: 31.03.2004 DE 102004016525; 28.06.2004 DE 102004031355
(43) Veröffentlichungstag der Anmeldung: 13.12.2006
(73) Patentinhaber: Schleifring und Apparatebau GmbH, 82256 Fürstenfeldbruck (DE)
(72) Erfinder: KRUMME, Nils, 82340 Feldafing (DE); LOHR, Georg, 82223 Eichenau (DE)
(74) Vertreter: Lohr, Georg
(86) Internationale Anmeldenummer: PCT/EP2005/003396
(87) Internationale Veröffentlichungsnummer: WO 2005/094687

(56) Entgegenhaltungen:
- US-A- 4 692 721
- US-A- 5 530 422
- US-B1- 6 181 766
- US-B1- 6 301 324
- US-B2- 6 580 853
- MEINKE H, GUNDLACH F W: "Taschenbuch der Hochfrequenztechnik S. 308-313" 1968, SPRINGER , BERLIN , XP002333224 das ganze Dokument

## Beschreibung

### Technisches Gebiet

Die Erfindung betrifft einen Drehübertrager zum Einsatz in Computertomografen. Hierbei erfolgt die Übertragung der von dem Röntgendetektor gewonnenen digitalen Bilddaten berührungslos zwischen der drehbaren Gantry und dem stationären Teil des Computertomografen. Weiterhin können auch Daten in der entgegengesetzten Richtung zu Steuerung der drehbaren Gantry übertragen werden.

### Stand der Technik

Aus der US 5,530,422 ist eine Vorrichtung zur berührungslosen Drehübertragung in Computertomografen bekannt. Darin wird das zu übertragende Signal auf der drehbaren Gantry in eine differentiell betriebene Streifenleitung eingespeist und durch eine kapazitive Sonde an dem stationären Teil abgegriffen. Derartige Vorrichtungen sind bis zu Datenraten in einer Größenordnung von ca. 1 GBaud einsetzbar. Diese Grenze kann mit weiterbildungen, wie sie beispielsweise in der US 6,181,766 offenbart sind, geringfügig nach oben verschoben werden. Hierzu werden geeignete Kodierungen bzw. Modulationsverfahren eingesetzt.

### Darstellung der Erfindung

Der Erfindung liegt die Aufgabe zugrunde, eine Vorrichtung zur kontaktlosen Übertragung digitaler Signale zwischen zwei gegeneinander beweglichen Einheiten und insbesondere zwischen dem stationären und dem rotierenden Teil eines Computertomografens gegenüber dem Stand der Technik dahingehend weiterzubilden, dass eine breitbandiger Signalübertragung mit geringerer Abstrahlung, höherer Störfestigkeit und geringerer Bitfehlerrate ermöglicht'wird.

Eine erfindungsgemäße Lösung dieser Aufgabe ist in den unabhängigen Patentansprüchen angegeben. Weiterbildungen der Erfindung sind Gegenstand der abhängigen Ansprüche.

Zur Vereinfachung der Darstellung wird nachfolgend mehrfach auf eine Übertragung von dem rotierenden Teil auf das feststehende Teil eines Computertomografen Bezug genommen. Selbstverständlich ist eine erfindungsgemäße Vorrichtung auch in umgekehrter Übertragungsrichtung einsetzbar. Ebenso kann eine erfindungsgemäße Vorrichtung auch in anderen Anwendungen zur Drehübertragung und ebenso zur linearen Übertragung zweier gegeneinander beweglichen Einheiten herangezogen werden.

Die Erfindung umfasst eine Vorrichtung zur kontaktlosen Übertragung elektrischer und insbesondere digitaler Signale. Die Übertragung erfolgt mittels dielelektrischer Wellenleiter, welche vorzugsweise als NRD (Non Radiating Dielectric) ausgebildet sind. So erzeugt ein Sender 10 am drehenden Teil (Rotor) elektrische Signale, welche durch einen ersten Leiterkoppler 11 in den dielektrischen Wellenleiter 12a, 12b eingekoppelt werden. Die Signale werden in dem dielektrischen Wellenleiter geführt und an dessen Ende durch eine Terminierung 13a, 13b absorbiert. Zur Auskopplung der in dem dielektrischen Wellenleiter geführten Signale ist am feststehenden Teil wenigstens ein Koppler 14 vorgesehen. Dieser Koppler 14 wird in der Nähe des dielektrischen Wellenleiters 12a, 12b bewegt. Er koppelt einen Teil der elektrischen Energie der Signale des dielektrischen Wellenleiters aus und überträgt diese an einen Empfänger 15 am feststehenden Teil (Stator). Beispielhaft kann der dieelektrische Wellenleiter in bekannter Weise auf einem metallischen Substrat angeordnet sein. Dabei ist die Anordnung derart dimensioniert, dass sich die elektromagnetische Welle aus dem zu übertragenen Signal in dem Dielektrikum, vorzugsweise mit nur einem einzigen Mode fortpflanzt. Alternativ hierzu könnte auch die Ausbreitung mit einer Oberflächenwelle im Dielektrikum erfolgen. Diese kann wiederum im Dielektrikum selbst angeregt werden. Die Ankopplung an den dielektrischen Wellenleiter erfolgt vorzugsweise durch Richtkoppler, eine angrenzende Wellenleiterstruktur, mittels einer Hohlleiterwelle, einer Leitung, vorzugsweise einer Streifenleitung, besonders bevorzugt einer Mikrostreifenleitung, oder aber auch über Prismen beziehungsweise eine Gitterstruktur des Wellenleiters.

In einer besonders vorteilhaften Ausgestaltung der Erfindung ist der Koppler 14 ein elektrischer Koppler, welcher Signale aus dem dielektrischen Wellenleiter derart auskoppelt, dass diese in einer Leitung, beispielsweise als TEM-Welle, geführt werden können. Hierzu wird der Koppler vorzugsweise selbst in Leitungstechnik aufgebaut.

In einer weiteren vorteilhaften Ausgestaltung der Erfindung umfasst der Koppler 14 selbst einen dielektrischen Wellenleiter 16. Mit diesem dielektrischen Wellenleiter verbunden ist nun ein zweiter Leiterkoppler 17 zur Umwandlung der in dem dielektrischen Wellenleiter 16 des Kopplers 14 geführten Signale in elektrische Signale, welche in einer Leitung, beispielsweise als TEM-Welle geführt werden können, vorgesehen.

In einer weiteren vorteilhaften Ausgestaltung der Erfindung ist der zweite Leiterkoppler 17 an einem Ende des dielektrischen Wellenleiters 16 angebracht. Gleichzeitig ist an dem anderen Ende des dielektrischen wellenleiters 16 eine Terminierung 18 zum reflexionsfreien Abschluss des dielektrischen Wellenleiters 16 vorgesehen.

Eine weitere vorteilhafte Ausgestaltung der Erfindung sieht vor, dass wenigstens einer der Abschlüsse 13a, 13b oder 18 als Abschlüsse in Form von dielektrischen Wellenleitern ausgeführt sind.

In einer anderen Ausgestaltung der Erfindung ist wenigstens einer der Abschlüsse 13a, 13b oder 18 als Leitungskoppler zur Umsetzung in eine Leitungswelle, beispielsweise eine TEM-Welle ausgebildet. Weiterhin ist ein elektrischer Absorber beziehungsweise Abschlusswiderstand zur Terminierung, d. h. zum reflexionsfreien Abschluss der elektrischen Welle vorgesehen und mit dem Leitungskoppler verbunden.

Eine weitere vorteilhafte Ausgestaltung der Erfindung umfasst die Ausbildung des dielektrischen Wellenleiters 12a, 12b als differentielles Leitersystem. Hierbei sind zwei dielektrische Wellenleiter 41, 42 parallel geführt, wobei diese mit einem differentiellen Signal beaufschlagt werden. Somit ist der Momentanwert eines Signals an einem bestimmten Punkt des ersten dielektrischen Wellenleiters 41 genau invers zu dem Momentanwert des Signals an dem entsprechenden Punkt des zweiten dielektrischen Wellenleiter 42. Somit ergibt die Summe der Signale an jeder Stelle der dielektrischen Wellenleiter 41 und 42 den Wert 0. Dies führt zu einer wesentlichen Reduzierung der elektromagnetischen Emissionen.

In einer weiteren vorteilhaften Ausgestaltung der Erfindung ist der Koppler 14 als differentieller Koppler ausgeführt. Dies bedeutet, dass in dem Koppler ausschließlich differentielle Signale zum Empfang ausgewertet werden. Gleichtaktsignale, welche beispielsweise durch eine externe Störung gleichmäßig in die beiden dielektrischen Wellenleiter 41 beziehungsweise 42 oder auch in die beiden Wellenleiter 43 beziehungsweise 44 des Kopplers 14 eingekoppelt werden, können somit unterdrückt beziehungsweise gedämpft werden. Somit haben derartige Störsignale keinen Einfluss mehr auf das Nutzsignal.

Eine weitere vorteilhafte Ausgestaltung der Erfindung sieht vor, dass in dem Sender 10 ein Modulator zur Anpassung der zu sendenden Daten an den dielektrischen Wellenleiter vorgesehen ist. Entsprechend ist in dem Empfänger 15 auch ein Demodulator zur Rückgewinnung der Daten vorgesehen. Ein solcher Modulator setzt den spektralen Bereich der zu übertragenden Daten in einen anderen spektralen Bereich um, welcher besser zur Übertragung durch den dielektrischen Wellenleiter geeignet ist. Die Modulation kann hier nach allen bekannten Verfahren, beispielsweise durch Mischung mit einem Träger, oder aber auch durch Kodierungen bzw. durch Kombination mehrerer Verfahren erfolgen. Entsprechend hierzu ist auch die Demodulation angepasst. Besonders effizient ist es, hier eine geeignete Kodierung des Signals einzusetzen, so dass die Bandbreite des zu übertragenden Signals innerhalb der übertragbaren Bandbreite des dielektrischen Wellenleiter liegt.

In einer weiteren vorteilhaften Ausgestaltung der Erfindung ist der dielektrische Wellenleiter 12a, 12b in wenigstens zwei Segmente unterteilt. Durch die Unterteilung in mehrere Segmente können beispielsweise mehrere Datenströme gleichzeitig übertragen werden. Hierzu sind unterschiedliche Datenströme in unterschiedliche Segmente einzuspeisen und gleichzeitig entsprechend mehrere Empfänger zum Empfang der unterschiedlichen Datenströme vorzusehen. Weiterhin kann auf Grund der Segmentierung die Länge der einzelnen dielektrischen Wellenleiter kürzer gehalten werden. Dies führt zu verringerten Verlusten innerhalb der dielektrischen Wellenleiter sowie zu einer erhöhten Störfestigkeit. Besonders vorteilhaft ist es, wenn insgesamt zwei Segmente des dielektrischen Wellenleiters vorgesehen sind, welche gleich lang sind. Weiterhin ist in dieser Ausführungsform ein gemeinsamer Ort der Signaleinkopplung vorgesehen, von dem sich die Signale in den Segmenten der dielektrischen Wellenleiter in entgegengesetzten Richtungen ausbreiten. Am Ende der dielektrischen Wellenleiter sind Terminierungen, beispielsweise in Form von Absorbern vorgesehen. Durch diese Ausgestaltung liegen an den Punkten der Annäherung der beiden Segmente, nämlich am Ort der Signaleinkopplung sowie am Ort der Terminierungen phasengleiche Signale an.

In eine andere Ausgestaltung der Erfindung ist der dielektrische Wellenleiter 12 in eine Vielzahl kurzer Leiter-Segmente unterteilt. Hierbei ist Vorteilhafterweise die Länge der Leitersegmente derart kurz gewählt, dass die Reflexionen in den einzelnen Segmenten noch zu keiner wesentlichen Beeinträchtigung der zu übertragenden Signale führen. Somit kann die Terminierung der einzelnen Segmente entfallen. Gerade bei Drehübertragern mit kleinen Durchmessern im Bereich einiger Zentimeter kann auch der dielektrischen Wellenleiter 12 ohne Terminierung eingesetzt werden. Ebenso kann er wahlweise auch als geschlossener Ring ausgeführt sein.

Eine weitere vorteilhafte Ausgestaltung der Erfindung sieht vor, dass wenigstens eine der Terminierungen 13a, 13b oder 18 in Form von Absorbern in dielektrischer Wellenleitertechnik ausgeführt sind.

Eine weitere Alternative der Erfindung sieht vor, dass wenigstens eine der Terminierungen 13a, 13b oder 18 als elektrische Abschlusswiderstände ausgebildet ist, wobei die Ankopplung über einen dielektrischen Leiterkoppler erfolgt.

Eine weitere vorteilhafte Ausgestaltung der Erfindung sieht vor, dass der Koppler 14 als Richtkoppler ausgeführt ist. Durch die Ausbildung eines Richtkopplers werden von diesem nur Signale aus dem dielektrischen Welleleiter 12a, 12b abgegriffen, welche sich in einer vorgegebenen Richtung ausbreiten. Somit können beispielsweise Störsignale, welche sich in entgegengesetzter Richtung ausbreiten, unterdrückt werden. Weiterhin erlaubt der Einsatz von Richtkopplern,auf einfache Art und Weise die Übertragung mehrerer Kanäle beziehungsweise eine bidirektionale Übertragung. Zur unidirektionalen Übertragung mehrerer Kanäle sind dielektrische Leiterkoppler 11 an unterschiedlichen Orten des dielektrischen Wellenleiters 12a, 12b anzubringen, so dass sich die unterschiedlichen Signale in entgegengesetzten Richtungen des gleichen Leiterzweigs fortpflanzen.

Eine weitere Vorteilhafte Ausgestaltung der Erfindung umfasst ein Lager, welches vorzugsweise in der Nähe eines Kopplers 14 zur Führung des Kopplers in einem definierten, geringen Abstand zum dieelektrischen Wellenleiter 12 vorgesehen ist. Dieses Lager und umfasst vorzugsweise ein hydrostatisches oder auch hydrodynamisches Lager und besonders bevorzugt ein Luftlager. Derartige Luftlager zeichnen sich durch einfache Konstruktion, große Robustheit und insbesondere eine hohe mechanische Steifigkeit aus.

Weiterhin ist Gegenstand der Erfindung ein Drehübertrager (Schleifring) zur Übertragung zwischen gegeneinander drehbaren Einheiten, welcher eine zuvor beschriebene Übertragungsstrecke basierend auf einem dielektrischen Wellenleiter umfasst. In einen solchen Schleifring kann erfindungsgemäß ein dielelektrischer Wellenleiter in verschiedenen vorteilhaften Ausführungsformen integriert werden. So kann er beispielsweise einstückig aus dem Material des Schleifrings selbst bestehen. Er könnte auch, ebenfalls aus demselben Material, von einem dünnen Steg, vorzugsweise in seinem Mitte gehalten, radial gesehen, innen oder außen am Schleifring angebracht sein. Ebenso könnte das Dielektrikum auch als mechanische Halterung für Schleifbahnen zur kontaktierenden elektrischen Übertragung dienen. Weiterhin könnte der dieelektrische Wellenleiter selbst auch aus zwei Teilen (vorzugsweise Hälften) bestehen, wobei ein Teil rotiert und das andere feststeht.

Ein anderer Gegenstand der Erfindung ist ein Computertomograph mit wenigstens einem der oben angegebenen Drehübertrager basierend auf einem dielektrischen Wellenleiter zur Datenübertragung. Hierbei kann die Übertragung selbstverständlich wahlweise von dem rotierenden Teil zum stationären Teil und umgekehrt erfolgen. Optional können zur Übertragung mehrerer Signale beziehungsweise zur Erhöhung der Übertragungsrate mehrere Drehübertrager eingesetzt werden.

Ein weiterer Gegenstand der Erfindung ist ein Verfahren zur Übertragung von Daten zwischen gegeneinander beweglichen Einheiten, wobei die Signale eines Senders in einem dielektrischen Wellenleiter entlang der Strecke der Bewegung geführt werden und an der Position eines Empfängers mittels eines Abgriffes ausgekoppelt werden. Alternativ hierzu können die Signale in dem dielektrischen Wellenleiter in einem begrenzten Bereich der Bewegung schräg zur Bahn der Bewegung übertragen werden.

Eine erfindungsgemäße Vorrichtung ist besonders einfach und preisgünstig realisierbar, da auch kostengünstige dielektrische Materialien wie Polyethylen (PE) einsetzbar sind. Weiterhin können zu Modulation Standard-Funkmodems eingesetzt werden. Da die Ausbreitung des elektromagnetischen Feldes auf das Dielektrikum des dielektrischen Wellenleiters beschränkt ist, werden im Vergleich zu Funklösungen wesentlich weniger elektromagnetische Störungen in die Umgebung abgegeben. Gleichzeitig ist das System selbstverständlich auch störunempfindlicher, da es nur die in dem dielektrischen Wellenleiter geführten Signale empfängt und auswertet. Durch die Führung der elektromagnetischen Signale in einem dielektrischen Wellenleiter können'problemlos mehrere gleichartige Übertragungseinrichtungen parallel in einem Computertomografen verwendet werden, ohne dass es hierbei zu Konflikten (Übersprechen) kommt. Auch ist ein "Abhören" der zu übertragenen Daten nicht möglich. Durch den Einsatz eines dielektrischen Wellenleiters ergibt sich im Vergleich zu Funkstrecken eine wesentlich höhere Bandbreite, da hier auch auf sonst nicht freigegebene Frequenzbänder zurückgegriffen werden kann. Auf Grund der wesentlich besseren Verkopplung durch den dielektrischen Wellenleiter ergibt sich im Empfänger ein wesentlich höherer Signalpegel und damit ein besserer Signal / Rauschabstand.

### Beschreibung der Zeichnungen

Die Erfindung wird nachstehend ohne Beschränkung des allgemeinen Erfindungsgedankens anhand von Ausführungsbeispielen unter Bezugnahme auf die Zeichnungen exemplarisch beschrieben.
Fig. 1 zeigt beispielhaft einen Computertomographen mit einer erfindungsgemäßen Vorrichtung.
Fig. 2 zeigt in schematischer Form eine erfindungsgemäße Vorrichtung.
Fig. 3 zeigt die Ankopplung eines Leitungskopplers an einen dielektrischen Wellenleiter.
Fig. 4 zeigt die Terminierung eines dielektrischen Wellenleiters.
Fig. 5 zeigt eine elektrische Ankopplung an einen dielektrischen Wellenleiter.
Fig. 6 zeigt den Aufbau eines Kopplers.
Fig. 7 zeigt eine erfindungsgemäße Vorrichtung im Schnitt.
Fig. 8 zeigt beispielhaft einen alternativen Aufbau eines dielektrischen Wellenleiters.
Fig. 9 zeigt beispielhaft einen alternativen Aufbau eines dielektrischen Wellenleiters.
Fig. 10 zeigt eine Integration eines dielektrischen Wellenleiters mit einem Schleifring.
Fig. 11 zeigt eine weitere Ausführungsform der Erfindung mit mehreren Sendern.
Fig. 12 zeigt eine weitere Ausführungsform der Erfindung mit einem einzigen Sender.

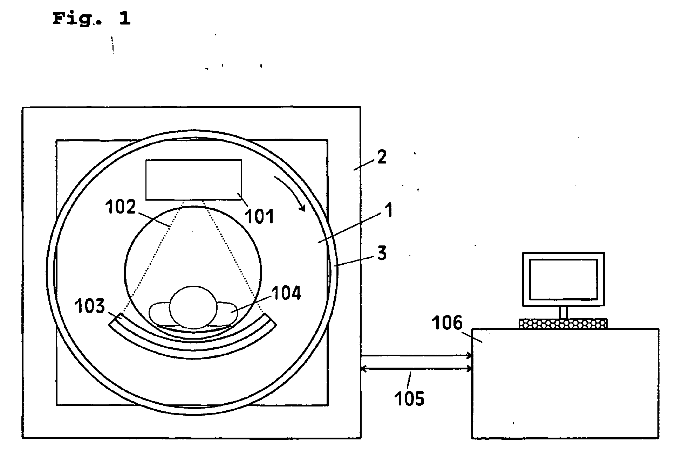

Fig. 1 zeigt beispielhaft eine erfindungsgemäße Vorrichtung. Der Computertomograf (CT-Scanner) besteht aus zwei mechanischen Hauptbestandteilen. Ein stationäres Teil 2 dient als Basis und Träger des ganzen Gerätes, in denen sich das rotierende Teil 1 dreht. Der Patient 104 wird auf einer Liege in der Öffnung des rotierenden Teils positioniert. Zur Abtastung des Patienten mittels Röntgenstrahlen 102 ist eine Röntgenröhre 101 sowie ein dieser gegenüberliegend angeordneter Detektor 103 vorgesehen. Röntgenröhre 101 und Detektor 103 sind auf dem rotierenden Teil 1 drehbar angeordnet. Ein Drehübertrager 3 dient zur elektrischen Verbindung zwischen dem rotierenden Teil 1 und dem stationären Teil 2. Hierbei werden einerseits die hohe elektrische Leistung zur Speisung der Röntgenröhre 101 in Richtung des rotierenden Teils 1 und gleichzeitig die Videodaten in der entgegengesetzten Richtung übertragen. Parallel hierzu ist eine Kommunikation von Steuerinformationen in beiden Richtungen vorgesehen. Eine Auswerte- und Steuereinheit 106 dient zur Bedienung des Computertomografen sowie zur Anzeige der erzeugten Bilder. Die Kommunikation mit dem Computertomografen erfolgt über eine bidirektionale Verbindung 105.

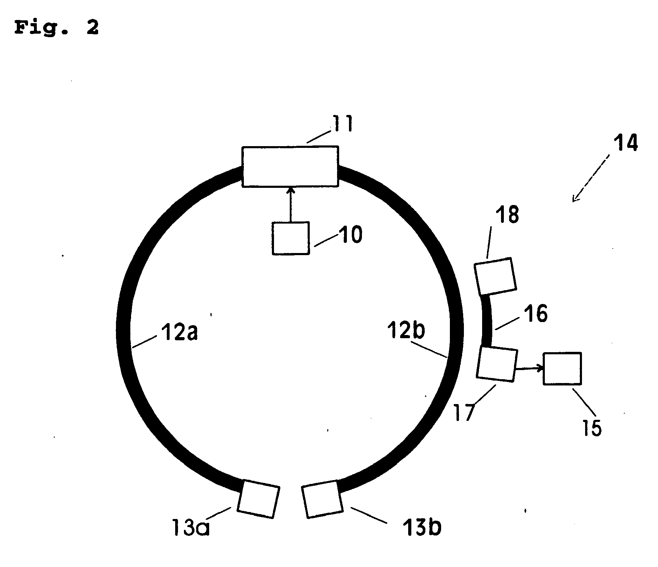

Fig. 2 zeigt beispielhaft eine erfindungsgemäße Vorrichtung im Detail. Ein Sender 10, beispielsweise der Röntgendetektor eines Computertomografen auf dessen drehender Gantry dient zur Aussendung von hochfrequenten Signalen in einen ersten Leitungskoppler 11 und von diesem in einen zweigeteilten dielektrischen Wellenleiter 12a, 12b. Dieser erste Leitungskoppler kann wahlweise in dielektrischer Wellenleiter-Technik oder auch rein elektrisch aufgebaut sein. An den Enden sind die Zweige des zweigeteilten dielektrischen Wellenleiters mittels der Terminierungen 13a, 13b weitgehend reflexionsfrei abgeschlossen. Relativ beweglich hierzu bzw. auf dem feststehenden Teil der Gantry angeordnet ist ein Empfänger 15, welcher die Signale, die von dem Koppler 14 abgegriffen werden, erhält. Der Koppler 14 weist vorzugsweise ebenfalls einen dielektrischen Wellenleiter auf und koppelt vorzugsweise das Feld in der Nähe des ersten dielektrischen Wellenleiters auf der Senderseite berührungslos aus. Dies kann beispielsweise durch die Verkopplung evaneszenter Felder erfolgen. Hierzu ist dann der dielektrischen Wellenleiter 16 als vorzugsweise gleichartiges Dielektrikum wie der dielektrische Wellenleiter 12a, 12b auszubilden und weiterhin sind beide mit einem möglichst geringen Spalt, vorzugsweise < Lambda/6 berührungslos oder unter Ausnutzung von Gleiteigenschaften gegeneinander zu führen. Dieser dielektrische Wellenleiter ist vorzugsweise ebenfalls mittels einer Terminierung 18 reflexionsfrei abgeschlossen. Der Leiterkoppler 11 muss nicht, wie hier dargestellt, unmittelbar an dem dielektrischen Wellenleiter 12a, 12b angeordnet sein. Vielmehr kann er auch entfernt angebracht und beispielsweise in den Sender 10 integriert sein. Die Signalverbindung zwischen dem Leiterkoppler 11 und dem dielektrischen Wellenleiter 12a, 12b erfolgt dann vorzugsweise durch verlängerte Enden des dielektrischen Wellenleiters 12a, 12b oder durch zusätzliche zwischengeschaltete dielektrischen Wellenleiter. Analoges gilt für den Leitungskoppler 17 und dem dielektrischen Wellenleiter 16.

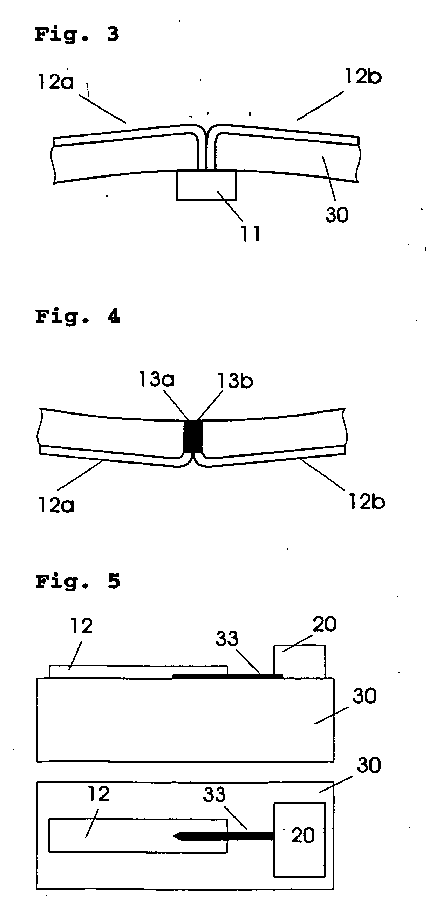

In Figur 3 ist beispielhaft die Anordnung mit einem zweigeteilten dielektrischen Wellenleiter 12a, 12b sowie einem ersten Leitungskoppler 11 auf dem mechanischen Träger 30 des Drehübertragers 3 dargestellt. In diesem Beispiel werden die Teile des dielektrischen Wellenleiters durch den Träger 30 an die Außenseite zu Abtastung durch den Koppler 14 geführt. Entsprechend umgekehrt wäre eine Ausführung zu gestalten, bei der der dieelektrische Wellenleiter auf der Innenseite des Trägers abgetastet wird.

In Figur 4 ist beispielhaft die Terminierung 13a, 13b des dieelektrischen Wellenleiters 12a, 12b dargestellt. Die Terminierung kann hier durch einen gemeinsamen Abschluss für beide Teile des dielektrischen Wellenleiters oder durch getrennte Terminierungen erfolgen.

Figur 5 zeigt eine beispielhafte elektrische Ankopplung eines elektrischen Senders 20 an den dielektrischen Wellenleiter 12 mittels einer Streifenleitung 33. Die Anordnung befindet sich auf einem elektrisch isolierenden Träger 30. Die Streifenleitung ragt in das Ende des dielektrischen Wellenleiters hinein bzw. liegt unter diesem und ist so ausgestaltet, dass eine möglichst reflexionsfreie Übertragung der elektrischen Signale erfolgt.

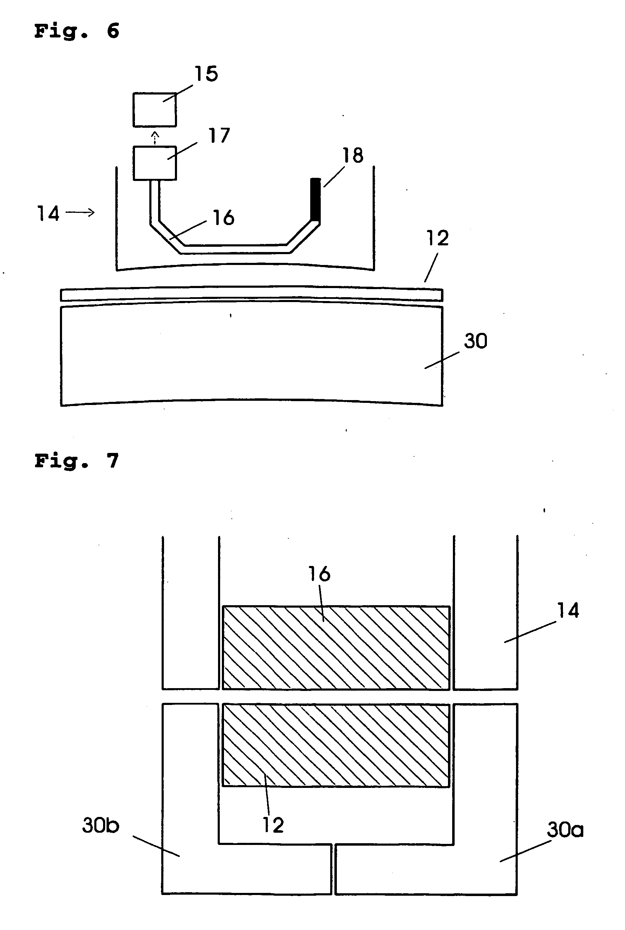

Figur 6 zeigt in seitlicher Ansicht einen Koppler 14, welcher über einem dielektrischen Wellenleiter 12 geführt wird. Der Koppler weist einen dieelektrische Wellenleiter 16 auf, welcher einen Teil des Signals des dielektrischen Wellenleiters 12 abgreift und diesen mittels eines zweiten Leitungskopplers 17 einem Empfänger 15 zuführt. Das andere Ende des dielektrischen Wellenleiters 16 ist mit der Terminierung 18 reflexionsfrei abgeschlossen.

In Figur 7 ist eine erfindungsgemäße Vorrichtung im Schnitt dargestellt. Ein dielektrischer Wellenleiter 12 ist beispielhaft in einem vorzugsweise aus einem elektrisch leitfähigen Material hergestellten zweigeteilten Träger 30a, 30b geführt. Zum Abgriff von Signalen aus dem dielektrischen Wellenleiter 12 ist ein Koppler 14 vorgesehen, welcher ebenfalls einen dielektrischen Wellenleiter 16 aufweist.

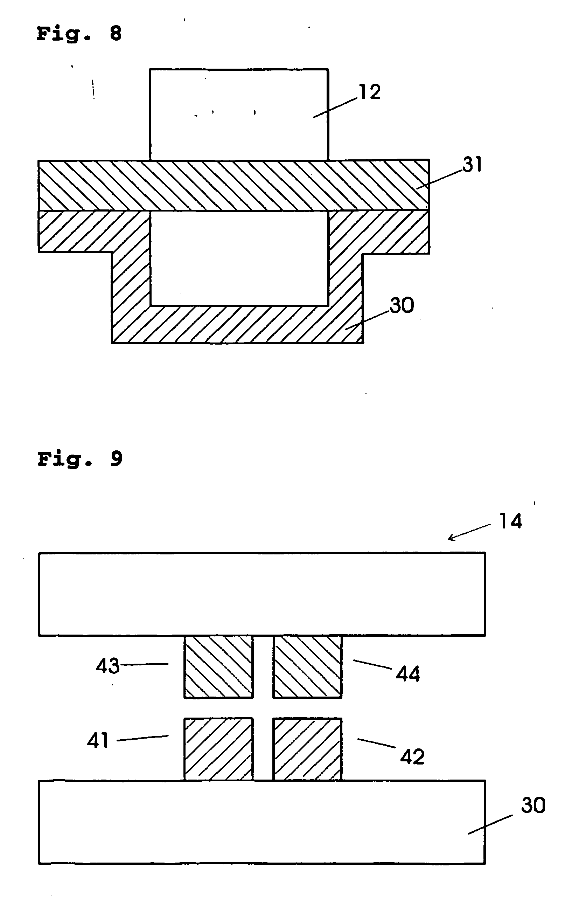

In Figur 8 ist noch eine weitere Ausführungsform eines dielektrischen Wellenleiters dargestellt. Ein elektrisch leitfähiger, vorzugsweise metallischer Träger 30 mit einer Aussparung wird von einer Trägerplatte 31 aus einem Dielektrikum abgedeckt. Auf dieser ist nun der dielektrische Wellenleiter 12 aufgebraucht.

Figur 9 zeigt eine differentielle Ausgestaltung der Erfindung, bei der der erste dielektrische Wellenleiter aus den beiden Teil-Wellenleitern 41 und 42 besteht. Die beiden Teil-Wellenleiter werden mit differentiellen Signalen gespeist. Dies bedeutet beispielsweise, dass die Summe der Signalamplituden der Teil-Wellenleiter 41 und 42 an der Schnittfläche der Zeichnung zu jedem Zeitpunkt gleich Null ist. Entsprechend ist vorteilhafterweise auch der Koppler differentiell mit den beiden Teil-Wellenleitern 43 und 44 aufgebaut.

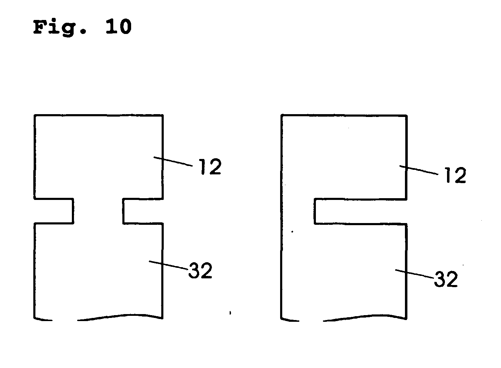

In Figur 10 ist die Integration eines dielektrischen Wellenleiters in einem Schleifring dargestellt. Ein mechanischer Schleifring-Körper 32 (Schleifring-Modul) trägt beispielsweise an der Außenseite den dielektrischen Wellenleiter 12. Selbstverständlich kann der dielektrische Wellenleiter auch an der Innenseite oder an der Fläche angeordnet sein. Besonders vorteilhaft ist es, wenn der Schleifring-Körper und der dieelektrische Wellenleiter aus demselben Material hergestellt werden können. Ein Verbindungssteg zur Halterung des dielektrischen Wellenleiters am Schleifring-Körper kann als separates Bauteil, aber auch besonders bevorzugt durch Bearbeitung des Schleifring-Körpers hergestellt werden. In diesem Beispiel ist der Verbindungssteg durch Ausdrehen (Einstich) hergestellt. Die Anordnung des Verbindungssteges ergibt sich aus den jeweiligen Moden der zu übertragenden elektromagnetischen Welle.

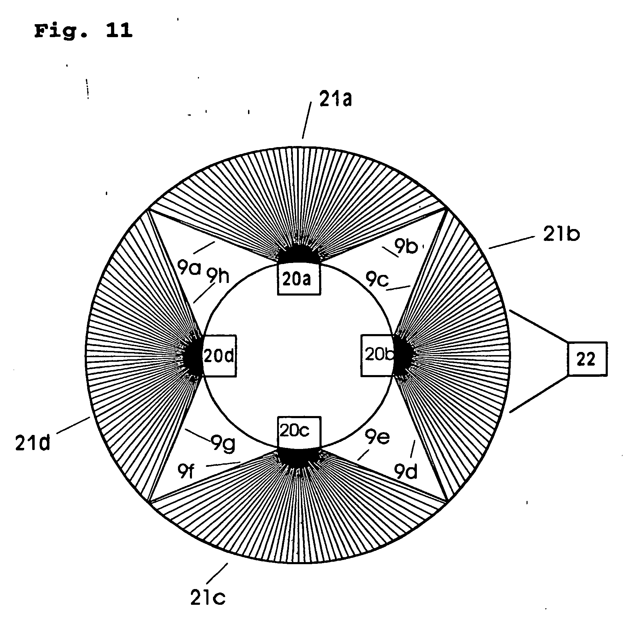

In Figur 11 ist eine weitere Ausführung der Erfindung dargestellt. Das Dielektrikum ist hierbei in mehrere Abschnitte 21a, 21b, 21c, 21d unterteilt. Hierzu dienen die Trennflächen 9a-9h. Diese Trennflächen können wahlweise durch metallisierte Lagen oder aber auch durch den Übergang in ein anderes Dielektrikum mit einer anderen Dielektrizitätskonstante realisiert sein. Sie dienen zur Führung bzw. Fokussierung der Signale der einzelnen Sender 20a, 20b, 20c, 20d. Diese Signale werden nun in dem zwischen den Trennflächen eingeschlossenen Dielektrikum geführt. Der Empfänger 22 ist gegenüber dieser Anordnung beweglich ausgeführt und kann beispielsweise wie eine Hornantenne mit oder ohne Dielektrikum ausgebildet sein. Er kann eine ähnliche Anordnung wie auf der Senderseite mit Dielektrikum und den Trennflächen aufweisen.

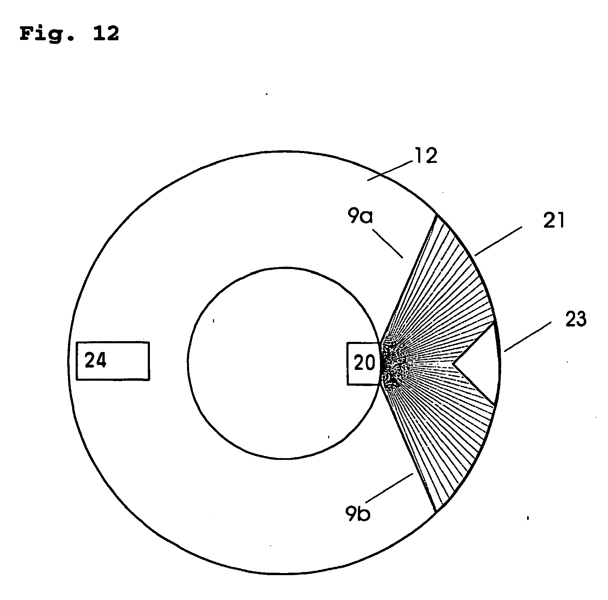

Figur 12 zeigt eine weitere Ausgestaltung der Erfindung, in welcher die Einkopplung der hochfrequenten Energie des Senders 20 in einen ringförmigen dielektrischen Wellenleiter 12 mittels eines Reflektors 23 erfolgt. Die Trennflächen 9a, 9b können zu Steuerung des Feldes eingesetzt werden. Die Vorrichtung ist aber auch ohne diese Trennflächen funktionsfähig. Grundsätzlich sei der ganze Innenraum zwischen den beiden kreisförmigen Begrenzungen mit einem Dielektrikum gefüllt. In diesem die Dielektrikum kann sich nun die von dem Sender abgestrahlte hochfrequente Energie kreisförmig ausbreiten. Ein Absorber 24 verhindert ein mehrfaches Umlaufen der elektromagnetischen Welle.

### Bezugszeichenliste

- 1: rotierendes Teil
- 2: stationäres Teil
- 3: Drehübertrager
- 9: Trennflächen
- 10: Sender
- 11: Erster Leitungskoppler
- 12: Dielektrischer wellenleiter (Rotor)
- 13: Terminierung
- 14: Koppler
- 15: Empfänger
- 16: Dielektrischer Wellenleiter (Stator)
- 17: Zweiter Leitungskoppler
- 18: Terminierung
- 20: Sender
- 21: Dielektrikum
- 22: Empfänger
- 23: Reflektor
- 24: Absorber
- 30: Träger
- 31: dielektrische Platte
- 32: Schleifring- Modul
- 33: Streifenleitung (Stripline)
- 41: Erster dielektrischer Wellenleiter einer differentiellen Anordnung
- 42: Zweiter dielektrische Wellenleiter einer differentiellen Anordnung
- 43: Erster dielektrische Wellenleiter eines differentiellen Kopplers
- 44: Zweiter dielektrische Wellenleiter eines differentiellen Kopplers
- 101: Röntgenröhre
- 102: Röntgenstrahlung
- 103: Detektor
- 104: Patient
- 105: bidirektionale Verbindung
- 106: Auswerte- und Steuereinheit

## Patentansprüche

1. Drehübertrager (3) für Computertomografen zur Übertragung von einem rotierendes Teil (1), umfassend einen Sender (10) zur Erzeugung elektrischer Signale, zu einem gegenüber diesem drehbar gelagerten stationären Teil (2), umfassend einen Empfänger (15) zum Empfang elektrischer Signale, dem rotierenden Teil (1) zugeordnet wenigstens ein Wellenleiter (12) gespeist von wenigstens einem ersten Leitungskoppler (11) zur Einkopplung der elektrischen Signale des Senders (10), und
dem stationären Teil (2) zugeordnet wenigstens ein Koppler (14) zum Abgriff von elektrischen Signalen des wenigstens einen Wellenleiters (12) und Weiterleitung der elektrischen Signale an einem Empfänger (15) vorgesehen ist, wobei
der Wellenleiter (12) in wenigstens zwei Segmente unterteilt ist, wobei die Segmente eine näherungsweise gleiche Länge aufweisen und durch einem ersten Leitungskoppler (11) Signale in die Segmente der Wellenleiter eingekoppelt werden, welche sich in entgegengesetzten Richtungen ausbreiten und die von dem Leitungskoppler entfernten Enden der Segmente mit Terminierungen (13) versehen sind, **dadurch gekennzeichnet, dass** der Wellenleiter ein dielektrischer Wellenleiter ist.

2. Vorrichtung nach Anspruch 1,
**dadurch gekennzeichnet, dass**
wenigstens einen Koppler (14) selbst einen dielektrischen Wellenleiter (16) umfasst, mit dem Signale aus wenigstens einem dielektrischen Wellenleiter (12) ausgekoppelt werden und weiterhin wenigstens einen zweiter Leiterkoppler (17) zur Umwandlung der Signale des dielektrischen Wellenleiters (16) in elektrische Signale, vorzugsweise als TEM-Welle vorgesehen ist, wobei diese Signale weiter an einen Empfänger (15) geführt werden.

3. Vorrichtung nach Anspruch 2,
**dadurch gekennzeichnet, dass**
an dem dielektrischen Wellenleiter (16) wenigstens eine Terminierung (18) zum reflexionsfreien Abschluss des dielektrischen Wellenleiters (16) vorgesehen ist, wobei vorzugsweise wenigstens eine der Terminierungen (18) an einem, dem zweiten Leitungskoppler (17) gegenüberliegenden Ende des dielektrischen Wellenleiters (16) angeordnet ist.

4. Vorrichtung nach Anspruch 1,
**dadurch gekennzeichnet, dass**
an wenigstens einem dielektrischen Wellenleiter (12) wenigstens eine Terminierung (13) zum reflexionsfreien Abschluss des dielektrischen Wellenleiters (12) vorgesehen ist, wobei vorzugsweise wenigstens eine der Terminierungen (13) an einem, dem ersten Leitungskoppler (11) gegenüberliegenden Ende des wenigstens einen dielektrischen Wellenleiters (12) angeordnet ist.

5. Vorrichtung nach Anspruch 3 oder 4,
**dadurch gekennzeichnet, dass**
wenigstens eine der Terminierungen (13, 18) als Abschluss in Form von verlustbehafteten dielektrischen Wellenleiter ausgeführt ist.

6. Vorrichtung nach Anspruch 3 oder 4,
**dadurch gekennzeichnet, dass**
wenigstens eine der Terminierungen (13, 18) einen weiteren Leitungskoppler zur Umsetzung der in dielektrischen Wellenleiter geführten Signale in eine Leitungswelle, vorzugsweise eine TEM-Welle aufweist und dieser mit einem reflexionfreien Abschluss zur Absorption der elektrische Energie verbunden ist.

7. Vorrichtung nach einem der vorhergehenden Ansprüche,
**dadurch gekennzeichnet, dass**
wenigstens ein dielektrischer Wellenleiter (12) als differentielles Leitersystem ausgebildet ist, welches zwei parallele dielektrische Wellenleitersegmente aufweist, welche mit einem differentiellen Signal beaufschlagt werden, wobei vorzugsweise auch der Koppler (14) ein differentielles Koppelsystem aufweist.

8. Vorrichtung nach einem der vorhergehenden Ansprüche,
**dadurch gekennzeichnet, dass**
dem Sender (10) zugeordnet ein Modulator zur Anpassung der zu sendenden Daten an die Übertragungseigenschaften des dielektrischen Wellenleiters (12) vorgesehen ist und weiterhin dem Empfänger (15) zugeordnet ein hierzu passender Demodulator zur Rückgewinnung der Signale vorgesehen ist.

9. Vorrichtung nach einem der vorhergehenden Ansprüche,
**dadurch gekennzeichnet, dass**
der dielektrische Wellenleiter (12) in eine Vielzahl von Segmenten unterteilt ist, wobei die Segmente vorzugsweise eine näherungsweise gleiche Länge aufweisen und die einzelnen Segmente keine Terminierungen (13) aufweisen.

10. Vorrichtung nach einem der vorhergehenden Ansprüche,
**dadurch gekennzeichnet, dass**
der Koppler (14) als Richtkoppler ausgeführt ist und somit in der Lage ist, die in dem dielektrischen Wellenleiter 12 geführten Signale richtungsselektiv zu empfangen.

11. Vorrichtung nach einem der vorhergehenden Ansprüche,
**dadurch gekennzeichnet, dass**
wahlweise wenigstens ein Erster Leitungskoppler (11) oder ein zweiter Leitungskoppler (17) als Richtkoppler ausgeführt ist.

12. Vorrichtung nach einem der vorhergehenden Ansprüche,
**dadurch gekennzeichnet, dass**
ein Lager, vorzugsweise ein hydrostatisches Lager, besonders bevorzugt ein Luftlager vorgesehen ist, um den Koppler (14) in einem definierten, geringen Abstand zum dielektrischen Wellenleiter (12) zu führen.

13. Vorrichtung nach einem der vorhergehenden Ansprüche,
**dadurch gekennzeichnet, dass**
wenigstens ein dielektrischer Wellenleiter (12) in einen Schleifring-Körper bzw. ein Schleifring-Modul integriert ist und vorzugsweise aus dem gleichen Material besteht.

14. Computertomograf mit einem rotierenden Teil (1) und einem stationären Teil (2) umfassend wenigstens einen Drehübertrager (3) nach einem der vorhergehenden Ansprüche zur Übertragung von Signalen wahlweise von dem rotierenden Teil (1) zu dem stationären Teil (2) beziehungsweise in umgekehrter Richtung.

15. Verfahren zur Übertragung von Signalen zwischen zwei gegeneinander drehbaren Teilen in Computertomographen mit einem Drehübertrager nach Anspruch 1, umfassend die folgenden Schritte: Einspeisen der elektrischen Signale eines Senders in wenigstens einen dielektrischen Wellenleiter, führen der elektrische Signale entlang der Bahn der Drehbewegung, auskoppeln der elektrischen Signale aus wenigstens einem dielektrischen Wellenleiter.

## Claims

1. Rotating data transmission device (3) for computer tomographs, for transmitting from a rotating part (1) including a transmitter (10) for generating electrical signals, to a stationary part (2) rotatably supported relative thereto and including a receiver (15) for receiving electrical signals,
at least one waveguide (12) that is assigned to the rotating part (1) and fed from at least one first line coupler (11) for coupling in electrical signals from the transmitter (10), and
at least one coupler (14) that is assigned to the stationary part (2), provided for tapping-off electrical signals from the at least one waveguide (12) and relaying the electrical signals to a receiver (15), wherein
the waveguide (12) is divided into at least two segments, the segments being of approximately the same length, and that signals are coupled into the segments of the waveguides through a first line coupler (11) and propagate in opposite directions, and that the ends of the segments distant from the line coupler are provided with terminations (13),
**characterized in that**
the waveguide is a dielectric waveguide.

2. Device according to claim 1,
**characterized in that**
at least one coupler (14) itself comprises a dielectric waveguide (16) for coupling out signals from at least one dielectric waveguide (12), and furthermore one second line coupler (17) is provided for converting the signals of the dielectric waveguide (16) to electrical signals, preferably as a TEM wave, these signals being further passed to a receiver (15).

3. Device according to claim 2,
**characterized in that**
at least one termination (18) for terminating the dielectric waveguide (16) in a manner free from reflection is provided on the dielectric waveguide (16), at least one of the terminations (18) preferably being disposed at an end of the dielectric waveguide (16) opposite to the second line coupler (17).

4. Device according to claim 1,
**characterized in that**
at least one termination (13) for terminating the dielectric waveguide (12) in a manner free from reflection is provided on at least one dielectric waveguide (12), preferably at least one of the terminations (13) being disposed at an end of the at least one dielectric waveguide (12) opposite to the first line coupler (11).

5. Device according to claim 3 or 4,
**characterized in that**
at least one of the terminations (13, 18) is adapted to be a termination in the form of a dielectric waveguide attended by losses.

6. Device according to claim 3 or 4,
**characterized in that**
at least one of the terminations (13, 18) comprises another line coupler for converting the signals carried in the dielectric waveguide to a line wave, preferably a TEM wave, and this line coupler is connected to a termination free from reflection to absorb the electrical energy.

7. Device according to any one of the preceding claims,
**characterized in that**
at least one dielectric waveguide (12) is designed to be a differential conductor system comprising two parallel dielectric waveguide segments to which a differential signal is applied, the coupler (14) preferably also comprising a differential coupling system.

8. Device according to any one of the preceding claims,
**characterized in that**
a modulator for matching the data to be transmitted to the transmission characteristics of the dielectric waveguide (12) is provided and assigned to the transmitter (10), and that furthermore a suitable demodulator for retrieving the signals is provided and assigned to the receiver (15).

9. Device according to any one of the preceding claims,
**characterized in that**
the dielectric waveguide (12) is divided into a multitude of segments, the segments preferably having approximately the same length, and the individual segments having no terminations (13).

10. Device according to any one of the preceding claims,
**characterized in that**
the coupler (14) is designed to be a directional coupler, and therefore able to receive the signals carried in the dielectric waveguide (12) in a directionally selective manner.

11. Device according to any one of the preceding claims,
**characterized in that**
optionally at least one first line coupler (11) or a second line coupler (17) is designed to be a directional coupler.

12. Device according to any one of the preceding claims,
**characterized in that**
a bearing, preferably a hydrostatic bearing, and most preferably an air bearing, is provided to guide the coupler (14) at a defined small distance from the dielectric waveguide (12).

13. Device according to any one of the preceding claims,
**characterized in that**
at least one dielectric waveguide (12) is incorporated into a slipring body or a slipring module and preferably consists of the same material.

14. Computer tomograph with a rotating part (1) and a stationary part (2), comprising at least one rotating data transmission device (3) according to any one of the preceding claims for transmitting signals optionally from the rotating part (1) to the stationary part (2), or in the opposite direction.

15. Method for transmitting signals between two parts that are rotatable relative to each other, in a computer tomograph, comprising the following steps: feeding the electrical signals from a transmitter into at least one dielectric waveguide, passing the electrical signals along the track of the rotary movement, coupling the electrical signals out of at least one dielectric waveguide.

## Revendications

1. Transmetteur rotatif (3) pour scanographes pour la transmission d'une partie rotative (1), comprenant un émetteur (10) destiné à générer des signaux électriques, vers une partie stationnaire (2) supportée avec possibilité de rotation vis-à-vis de celle-ci, comprenant un récepteur (15) pour la réception de signaux électriques,
dans lequel est prévu au moins un guide d'ondes (12) associé à la partie rotative (1) et alimenté par au moins un coupleur de ligne (11) pour le couplage des signaux électriques de l'émetteur (10), et
au moins un coupleur (14) associé à la partie stationnaire (2) pour capter des signaux électriques de l'au moins un guide d'ondes (12) et transmettre les signaux électriques à un récepteur (15),
le guide d'ondes (12) étant divisé en au moins deux segments, lesquels segments ont une longueur approximativement égale, et des signaux étant couplés dans les segments du guide d'ondes par un premier coupleur de ligne (11), lesquels signaux se propagent dans des directions opposées, et les extrémités des segments distantes du coupleur de ligne étant munies de terminaisons (13),
**caractérisé en ce que** le guide d'ondes est un guide d'ondes diélectrique.

2. Dispositif selon la revendication 1, **caractérisé en ce qu'**au moins un coupleur (14) comprend lui-même un guide d'ondes diélectriques (16) avec lequel les signaux sont découplés à partir d'au moins un guide d'ondes diélectrique (12), et il est prévu au moins un deuxième coupleur de guide d'ondes (17) pour convertir les signaux du guide d'ondes diélectrique (16) en signaux électriques, de préférence sous la forme d'une onde TEM, ces signaux étant en en outre transmis à un récepteur (15).

3. Dispositif selon la revendication 2, **caractérisé en ce qu'**il est prévu sur le guide d'ondes diélectrique (16) au moins une terminaison (18) pour la terminaison sans réflexion du guide d'ondes diélectrique (16), au moins une des terminaisons (18) étant de préférence disposée à une extrémité du guide d'ondes diélectrique (16) située vis-à-vis du deuxième coupleur de ligne (17).

4. Dispositif selon la revendication 1, **caractérisé en ce qu'**il est prévu sur au moins un guide d'ondes diélectrique (12) au moins une terminaison (13) pour la terminaison sans réflexion du guide d'ondes diélectrique (12), au moins une des terminaisons (13) étant de préférence disposée sur une extrémité de l'au moins un guide d'ondes diélectrique (12) située vis-à-vis du premier coupleur de ligne (11).

5. Dispositif selon la revendication 3 ou 4, **caractérisé en ce qu'**au moins une des terminaisons (13, 18) est réalisée comme une terminaison sous la forme de guides d'ondes diélectriques affectés par des pertes.

6. Dispositif selon la revendication 3 ou 4, **caractérisé en ce qu'**au moins une des terminaisons (13, 18) comporte un autre coupleur de ligne pour la conversion des signaux acheminés dans le guide d'ondes diélectrique en une onde de guide, de préférence une onde TEM, et celui-ci est relié à une terminaison sans réflexion pour l'absorption de l'énergie électrique.

7. Dispositif selon l'une des revendications précédentes, **caractérisé en ce qu'**au moins un guide d'ondes diélectriques (12) est conçu comme un système de guide d'ondes différentiel qui comporte deux segments de guide d'ondes diélectrique parallèles recevant un signal différentiel, le coupleur (14) étant de préférence lui aussi un système de couplage différentiel.

8. Dispositif selon l'une des revendications précédentes, **caractérisé en ce qu'**il est prévu associé à l'émetteur (10) un modulateur pour l'adaptation des données transmises aux propriétés de transmission du guide d'ondes diélectrique (12) et il est prévu en outre associé au récepteur (15) un démodulateur adapté à celui-ci pour la récupération des signaux.

9. Dispositif selon l'une des revendications précédentes, **caractérisé en ce que** le guide d'ondes diélectrique (12) est divisé en une pluralité de segments, les segments ayant une longueur approximativement égale et les différents segments ne présentant pas de terminaisons (13).

10. Dispositif selon l'une des revendications précédentes, **caractérisé en ce que** le coupleur (14) est conçu comme un coupleur directionnel et peut ainsi recevoir les signaux transmis au guide d'ondes diélectrique (12) sélectivement en fonction de la direction.

11. Dispositif selon l'une des revendications précédentes, **caractérisé en ce qu'**au moins un premier coupleur de ligne (11) ou un deuxième coupleur de ligne (17) au choix est conçu comme un coupleur directionnel.

12. Dispositif selon l'une des revendications précédentes, **caractérisé en ce qu'**il est prévu un palier, de préférence un palier hydrostatique, en particulier un palier pneumatique, pour guider le coupleur (14) à une distance réduite et définie du guide d'ondes diélectrique (12).

13. Dispositif selon l'une des revendications précédentes, **caractérisé en ce qu'**au moins un guide d'ondes diélectrique (12) est intégré dans un corps de bague collectrice ou un module de bague collectrice et se compose de préférence du même matériau.

14. Scanographe avec une partie rotative (1) et une partie stationnaire (2) comprenant au moins un transmetteur rotatif (3) selon l'une des revendications précédentes pour la transmission de signaux de la partie rotative (1) à la partie stationnaire (2) ou en sens inverse au choix.

15. Procédé pour la transmission de signaux entre deux parties rotatives l'une par rapport à l'autre dans des scanographes avec un transmetteur rotatif selon la revendication 1, comprenant les étapes suivantes : fourniture des signaux électriques d'un émetteur à au moins un guide d'ondes diélectrique, guidage des signaux électriques le long de la trajectoire du mouvement rotatif, découplage des signaux électriques à partir d'au moins un guide d'ondes diélectrique.
